# EUROPEAN PATENT SPECIFICATION

(11) **EP 1 925 961 B1**
(45) Date of publication and mention of the grant of the patent: **29.03.2017**
(21) Application number: 08000029.2
(22) Date of filing: 30.06.1997
(51) Int. Cl.: G02B 21/00, G01N 21/47, A61B 5/00

(54) **Confocal imaging through thick dermal tissues**
Konfokale Abbildung durch dickes Hautgewebe
Imagerie confocale par des tissus dermiques épais

(43) Date of publication of application: 28.05.2008
(62) Divisional of application: 97933239.2
(73) Proprietor: LUCID, INC., Rochester, NY 14623 (US)
(72) Inventor: Zavislan, James M., Pittsford NY 14534 (US); Eastman, Jay M., Pittsford NY 14534 (US)
(74) Representative: Baldus, Oliver

(56) References cited:
- WO-A-92/17806
- WO-A-92/19930
- WO-A-96/21938
- WO-A1-96/39925
- DELANEY P M ET AL: "FIBER-OPTIC LASER SCANNING CONFOCAL MICROSCOPE SUITABLE FOR FLUORESCENCE IMAGING" APPLIED OPTICS,US,OPTICAL SOCIETY OF AMERICA,WASHINGTON, vol. 33, no. 4, 1 February 1994 (1994-02-01), pages 573-577, XP000429124 ISSN: 0003-6935

## Description

### Field of The Invention

The present invention relates to handheld confocal imaging system for in vivo clinical examinations of dermal and subdermal tissues using non-ionizing radiation, and particularly laser radiation which is of a wavelength capable of penetrating into the skin.

The invention is especially suitable for providing an instrument for dermal pathology applications. The invention is also applicable for visualizing sections in other scattering media than tissue. The invention enables the use of a laser as a source of illumination. The instrument may provide data to image processing computers, which may be programmed to provide high resolution images of dermal sections.

### Background of the Invention

Systems have been proposed for viewing the surface areas of the skin or the external surfaces of internal tissue. Viewing without scanning is described in Pennypacker, U.S. Patent 4,817,622, issued April 4, 1989 . Examination of internal tissue surfaces by means of beam scanning are proposed in Harris, U.S. Patent 5,120,953, issued June 9, 1992 , Ohki, U.S. Patent 5,122,653 issued June 16, 1992, Webb, U.S. Patent 4,768,874 issued September 6, 1988 and Pflibsen, U.S. Patent 4,991,953 issued February 12, 1991 . Such proposals have not provided a handheld instrument which is readily usable by a surgeon in clinical examinations for imaging the epidermis and dermis, especially in vertical sections or in horizontal sections at desired depths below the surface of the skin.

Document WO 96/39925 relates to a system and method for locating anatomical structures within biological tissue. A confocal imaging system is focused at a particular depth of interest. Light from different depths and different positions is rejected by use of a collimator at the focal point of the optics. The image is then built up by raster-scanning the object to be imaged.

Document WO 92/17806 relates to a scanning optical microscope instrument which provides a selection of optical paths with enhanced contrast of an image of an object by concentration of selected illumination.

Document WO 96/21938 a confocal microscope for generating a video-rate, real-time image of a sample.

Document WO 92/19930 relates to optical imaging, including utilizing such images to perform precision measurements on biological and other samples.

Document "Fiber-optic laser scanning confocal microscope suitable for fluorescence imaging", Peter M. Delaney, Martin R. Harris, and Roger G. King, Applied Optics, Vol. 33, Issue 4, pp. 573-577 (1994), relates to a fiber-optic laser scanning confocal microscope that utilizes a single-mode optical fiber as both the source and the detection aperture.

### Summary of the Invention

Accordingly, it is the principal object of the present invention to provide and improve clinical dermatological imaging system.

It is another object of the invention to provide an improved confocal imaging system which provides images of dermatological tissues and avoids the need for biopsies to detect the location of such abnormalities as basal cell carcinomas and melanomas.

It is a still further object of the present invention to provide an improved confocal dermatological imaging system which does not require ionizing radiation and may use a laser beam.

It is a still further object of the present invention to provide an improved confocal imaging system which provides in vivo imaging of dermatological tissue both at and below the skin and which may be handheld and which is capable of operating in various scattering media.

It is a still further object of the present invention to provide an improved confocal dermatological imaging system which may use a computer to generate images from data produced by the optics which provides confocal imaging and to display or provide images for further evaluation or computer enhancement.

### Brief Description Of The Drawings

The foregoing objects, features and advantages of the invention will become more apparent from a reading of the following description in connection with the accompanying drawings in which:
FIG. 1 is schematic diagram of a confocal imaging system embodying the invention;
FIG. 1a is a plan view of the head of the system shown in FIG. 1;
FIG. 2 is a block diagram of the system shown in FIG. 1, and especially the computer control and imaging system for acquisition and processing of the optical image;
FIG. 3 is a schematic diagram of the handheld confocal imaging system of FIG. 2 in use.

### Detailed Description Of The Invention

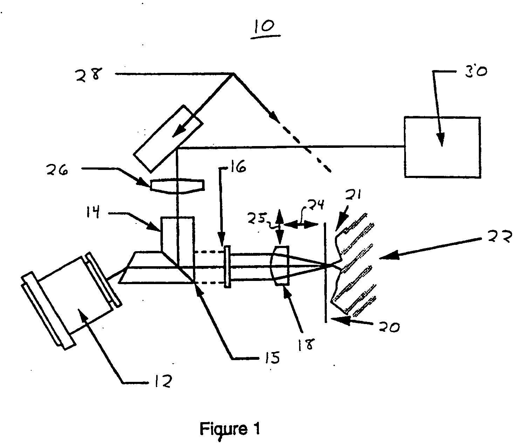

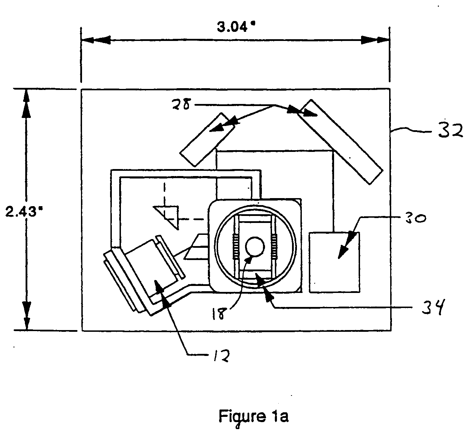

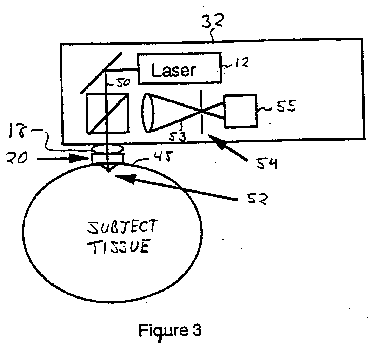

Referring to FIG. 1 there is shown a system is for in vivo diagnosis of dermatological tissues. The system 10 may be embodied in a handheld head 32 as shown in FIG. 1a and schematically in FIG. 3.

Referring more particularly to FIG. 1 there is shown a system 10 (or instrument) which contains optics of the type which are used in optical data storage heads which are used in recording and reading optical disks. Light from a laser diode, contained in a laser and collimator assembly 12, is collimated by a diffraction limited lens in the assembly 12 and is incident at an oblique angle on a beam splitter assembly 14. Refraction at this oblique angle causes the elliptical laser diode beam to become circular in cross-section. The circular beam passes through the beam splitter assembly 14 and a quarter wave plate 16 and is focused into the tissue 22 via a contact window 20 (a glass window plate) spaced from the sample, specimen or tissue 22 being examined, preferably by an optical contact liquid 21. In the event the sample is viscus or liquid, it may be located in a sample well (not shown).

The circular beam which passes through the beam splitter assembly 14 and the quarter wave plate 16 is focused into the sample by a precision focusing lens 18, which suitably has a numerical aperture of 0.5 and a focal length of 4.3 millimeters. These dimensions and parameters are exemplary and demonstrate that the optical system 10 may be miniaturized so as to be adapted to be handheld.

The quarter wave plate 16 converts the incident linear polarization from the laser in assembly 12 to circular polarization, i.e., the quarter wave plate is oriented 45° to the incident polarization. In other words, the beam from plate 16 is circularly polarized. The focusing lens 18 is movable both in a direction along its optical axis and laterally as indicated by the arrows 24 and 25, respectively. Position mechanical actuators 34 (FIG. 1a) may be used for moving the lens 18, and thereby control position of the focus spot of beam in the sample. These actuators 34 may be similar to those used in optical disk systems. The lens 18 may be mounted on a pair of such mechanical actuators. The actuators 34 provide lateral and vertical scanning of the focused laser beam in the tissue sample.

The focusing lens 18 also collects scattered light reflected from the sample. The amount of coherent light scattered back into the detection system (which includes lens 18, plate 16 and assembly 14) depends upon local variations of the refractive index and the absorption in the immediate neighborhood of the focus spot. This coherent light may be defined as the component of the reflected light having a circular polarization orthogonal to the polarization of the beam focused into the tissue sample. The scattered light is incident to plate 16 and then to beam splitter assembly 14. The plate 18 converts the coherent component of the scattered light into linear polarization, where beam splitter assembly 14 directs by reflection (or filters) the coherent light component of the scattered light at the beam splitting surface 15 in the beam splitter assembly 14. The reflected light passes through a relay lens 26. The light from relay lens 26 may be reflected from a pair of fold mirrors 28 (See also FIG. 1a). These fold mirrors 28 may be part of the beam splitter assembly 14. The relay lens 26 may also be part of this assembly 14.

The scanned light from the focus spot is reflected from the fold mirrors 28 to a pinhole photodetector assembly 30, which may also be considered part of the detection system. The fold mirrors 28 are used to make the instrument more compact. A prism assembly may alternatively be used, which is part of the beam splitting assembly 14, and allows the samples to be placed face down. This orientation allows gravity to assist in maintaining the sample in a stable viewing position. Maintaining a stable viewing position is also enhanced by the use of the window 20 as shown in FIG. 1.

A top view of the instrument is illustrated in FIG. 1a. Typical dimensions are given in FIG. 1a to illustrate the compacted size of the confocal imaging head 32. The elements in the head 32 may be located on a single board to provide unitized construction. The height of the head may be approximately two inches from the base to the nominal focal point of the focusing lens 18.

By scanning using the mechanical actuators 34 successive lines may be scanned at successive depths to provide images of vertical sections (i.e., along a vertical plane through the tissue sample). If desired the images may be formed from horizontal sections (i.e., along a horizontal plane through the tissue sample) as the lines are scanned horizontally. By tilting the sample, sections at desired angles to the surface of the sample (i.e., along a tilted or non-perpendicular plane) may be formed, such sections may also be formed by moving the lens 18 via actuator 34 as desired angles.

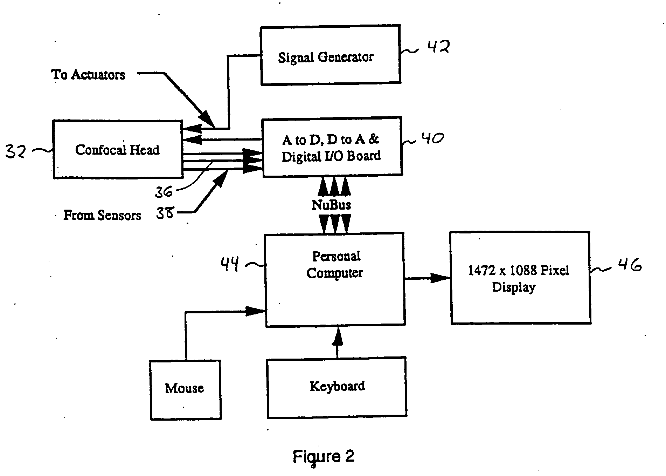

Referring to FIG. 2, there is shown a block diagram of the data acquisition and analysis system which is part of the imaging system 10 provided by the invention. The confocal head 32 is the head shown in FIGS. 1 and 1a. The output 36 from the head 32 is the output from the pinhole detector assembly 30. This output 36 is the confocal detector signal. Signals are also provided from sensors 38, namely a lateral position sensor and a vertical position sensor. These signals after amplification and filtering are acquired by a analog to digital converter of a digital I/O board 40. This board 40 may also be on a board with a circuit which provides a digital to analog channel to drive the lateral motion actuator. The vertical scanning actuator is driven from a signal derived from a conventional signal generator 42. The A to D, D to A and digital I/O board 40 is controlled and data is acquired via software in a personal computer 44, such as a Macintosh Quadra 950. Conventional software packages may be used for image analysis and for driving a display 46, which is shown by way of example as a 1472 by 1088 pixel display.

Referring to FIG. 3, there is shown the confocal imaging head 32 contacted against the skin 48 of a subject specimen using a mineral oil as an optical index matching fluid, which is an optical contact liquid 21 (FIG. 1) for reducing undesired reflections of light from the surface of the skin. The force against the skin 48 will be limited to that required to press the skin against the contact window 20 of the head 32. A laser beam 50 which may be relatively low power (e.g.,
6.3 milliwatts of optical power) is focused into the dermis of the specimen. The laser is operated at a wavelength capable of penetrating into the skin of the specimen, thus the skin may be considered transparent to the laser wavelength (or in other words, the skin is permeable to electromagnetic radiation of specified frequencies). The depth of focal point or spot 52 is varied from the surface of the stratum corneum to a few millimeters below the surface of stratum corneum. The nominal beam spot size may be, for example, 2.5 micrometers, full width half maximum. The laser spot is scanned laterally across the skin, for example at a rate of 3 to 10 hz. Different laser wavelengths may be selectively used for different resolution. Inasmuch as the energy delivered is proportional to the illuminating flux focused divided by the diameter of the spot, the scan length and the scan rate or frequency, the amount of incident flux is sufficiently low that damage to the specimen is avoided. The light scattered by the tissue is collected and the lights coherent component is re-imaged onto the pinhole aperture 54 of assembly 30, as shown in FIGS. 1 and 1a. The pinhole 54 transmits the coherent light from the focal region of the incident beam 53 to the detector 55 (of assembly 30) where it converts the light into an electrical signal. As the lens 18 scans laterally, the electrical signal is acquired by the computer and stored. Each scan represents a one dimensional trace of the reflectivity and scattering cross section of the dermis at a given level below the surface of the skin 48. A series of scans are made with the focal point positioned at progressively lower depths thereby providing a vertical cross section image of the skin which may be similar to a B-scan ultrasound image. As stated earlier, these scans may also be horizontal to provide a horizontal cross-section, or at an angle to provide an angular cross-section of the skin.

From the foregoing description it will be apparent that there has been provided an embodiment of a confocal imaging system for dermatological pathology applications. Variations and modifications of the herein described system and other applications for the invention will undoubtedly suggest themselves to those skilled in the art. Accordingly, the foregoing description should be taken as illustrative and not in a limiting sense.

## Claims

1. A method for confocal imaging a tissue sample having an exposed surface comprising the steps of:
providing a housing adapted to be handheld having a laser illumination source and an objective lens;
placing said tissue sample against a window attachable to said housing defining a tissue contacting surface in direct pressure contacting relationship with the surface of said tissue sample, so as to maintain a stable viewing position when said tissue sample is imaged via said objective lens and said housing is handheld, and said window is a plate of material transparent to said illumination;
focusing the illumination with said objective lens on or below the exposed surface to a focal spot;
scanning the focal spot in a plane through the tissue sample extending below said exposed surface;
collecting returned light from said tissue sample via said objective lens;
detecting the returned light via a pinhole aperture, in which said returned light is optically coupled to the pinhole aperture;
forming an image of a tissue section from said detected returned light; and displaying said image of said tissue section to avoid the need for biopsy to view cellular abnormalities in the tissue sample.

2. The method according to Claim 1 wherein said detecting step further comprises converting the returned light into electrical signals, and said method further comprises the steps of processing said electrical signals to provide an image of said tissue section.

3. The method as set forth in any of the preceding claims wherein said focusing, scanning, collecting, and detecting steps are carried out in said housing.

4. The method as set forth in any of the preceding claims wherein said scanning step is carried out with the aid of actuators.

5. The method as set forth in any of the preceding claims wherein said scanning step is carried out by moving said objective lens.

6. The method as set forth in any of the preceding claims wherein said tissue section represents one of a horizontally spaced section, angularly spaced section, or vertically spaced section.

7. The method as set forth in any of the preceding claims further comprising the step of converting the illumination from said source into circularly polarized light to enable said tissue sample to be illuminated by said circularly polarized light focused by said objective lens.

8. The method according to Claim 7 wherein said collecting step comprises the step of
collecting circularly polarized reflected light from the illuminated tissue sample which is orthogonal to the circularly polarized light which illuminated the tissue sample, and/or wherein preferably said converting step is carried out utilizing a quarter wave plate.

9. The method according to Claim 1 wherein said method further comprises the steps of:
providing a beam splitter for receiving said illumination from said source at an oblique angle to provide a circular beam; and polarizing said illumination from said beam slitter using a quarter wave plate to provide a circularly polarized beam, wherein said focusing step further comprises focusing said circularly polarized beam with said objective lens to the tissue sample, said receiving step further comprises receiving said returned light incident upon said quarter wave plate and then to said beam splitter, and said detecting step further comprises detecting a reflected part of said returned light incident from said beam splitter to a detector which detects returned light via said pinhole aperture.

10. The method according to Claim 9 wherein said returned light collected by said objective lens has a component which is circularly polarized orthogonal to the beam focused into the tissue sample, and said quarter wave plate converts the component of the returned light into linearly polarized orthogonal light, and said beam splitter, by reflecting part of said returned light, filters said components from said returned light.

11. A system for confocal imaging tissue samples having an exposed surface, comprising:
a housing which is adapted to be handheld, confocal imaging optics in said housing, said confocal imaging optics comprising,
a window attached to said housing defining a tissue contacting surface in direct pressure contacting relationship with a surface of said tissue sample, so as to maintain a stable viewing position when said housing is handheld and said tissue sample is imaged by said objective lens, said window is a plate of material transparent to said illumination,
an objective lens of said microscope and being adapted for focusing on or below the exposed surface and collecting returned light from the tissue sample,
actuators in said housing driven by electrical signals for scanning said focal spot through the tissue sample in which said focal spot extends below said exposed surface,
a diode laser in said housing which illuminates the tissue sample through confocal imaging optics,
a quarter wave plate in said housing converting an incident polarization from the diode laser,
a pinhole photodetector assembly in said housing which receives said returned light collected by said objective lens and converts said returned light into electrical signals,
a computer system which provide one or more images of tissue sections in accordance with the electrical signals outputted by said photodetector assembly, and
a display coupled to said computer system to display said one or more images of tissue sections and avoids the need for biopsy to detect any cellular abnormalities in the tissue sample.

12. The system according to Claim 11 further comprising mirrors which optically couple the returned light to said pinhole photodetector assembly.

13. The system according to Claim 11 further comprising means for processing said electrical signals from said photodetector assembly to enable said computer system to provide said one or more images.

14. The system according to Claim 11 wherein said confocal imaging optics, pinhole photodetector assembly and said laser diode are in a unitized construction in said housing.

15. The system according to Claim 11 wherein said one or more images of tissue sections each represents one of a horizontally spaced section, angularly spaced section, or vertically spaced section.

16. The system according to Claim 11 wherein said confocal said quarter wave plate converts said laser illumination into circularly polarized light which is focused by said objective lens to the tissue sample, and said objective lens collects said circularly polarized reflected light from the tissue sample which is orthogonal to the circularly polarized light which was focused by said objective lens to the tissue sample.

17. The system according to Claim 11 wherein said confocal imaging optics comprises: a beam splitter for receiving said laser illumination from said laser diode at an oblique angle to provide a circular beam, which is polarized by said quarter wave plate to provide a circularly polarized beam, wherein said objective lens is adapted to focus said circularly polarized beam and to collect light returned from said tissue sample, said returned light being incident to said quarter wave plate and then to said beam splitter, and said beam splitter reflects part of said returned light incident thereto, and said reflected part of said returned light is optically coupled said photodetector assembly.

18. The system according to Claim 17 wherein said returned light collected by said objective lens has a component which is circularly polarized orthogonal to the beam focused into the tissue sample, and said quarter wave plate converts the component of the returned light into linearly polarized orthogonal light, and said beam splitter, by reflecting part of said returned light, filters said components from said returned light.

## Patentansprüche

1. Verfahren zum konfokalen Abbilden einer Gewebeprobe mit einer freiliegenden Oberfläche, umfassend:
Bereitstellen eines Gehäuses, das so ausgelegt ist, dass es in der Hand gehalten wird, eine Laserbeleuchtungsquelle und eine Objektivlinse aufweist;
Platzieren der Gewebeprobe an einem Fenster, das an dem Gehäuse anbringbar ist und eine Gewebekontaktfläche in einer direkten Druckkontaktbeziehung mit der Oberfläche der Gewebeprobe definiert, damit eine stabile Betrachtungsposition aufrechterhalten wird, wenn die Gewebeprobe über die Objektivlinse abgebildet wird und das Gehäuse in der Hand gehalten wird, und das Fenster eine Platte aus einem Material ist, das für die Beleuchtung durchlässig ist;
Fokussieren der Beleuchtung mit der Objektivlinse auf oder unter der freiliegenden Oberfläche auf einen Brennpunkt;
Scannen des Brennpunkts in einer Ebene durch die Gewebeprobe, die unter der freiliegenden Oberfläche verläuft;
Sammeln von zurückgesandtem Licht von der Gewebeprobe über die Objektivlinse;
Erfassen des zurückgesandten Lichts über eine Lochblende, wobei das zurückgesandte Licht optisch in die Lochblende eingekoppelt wird;
Erzeugen eines Bildes eines Gewebeschnitts aus dem erfassten zurückgesandten Licht; und Anzeigen des Bildes des Gewebeschnitts, damit keine Biopsie notwendig ist, zum Betrachten von Zellanomalien in der Gewebeprobe.

2. Verfahren nach Anspruch 1, wobei der Schritt des Erfassens ferner ein Umwandeln des zurückgesandten Lichts in elektrische Signale umfasst und das Verfahren ferner die Schritte zum Verarbeiten der elektrischen Signale umfasst, damit ein Bild des Gewebeschnitts geliefert wird.

3. Verfahren nach einem der vorhergehenden Ansprüche, wobei die Schritte des Fokussierens, Scannens, Sammelns und Erfassens in dem Gehäuse durchgeführt werden.

4. Verfahren nach einem der vorhergehenden Ansprüche, wobei der Schritt des Scannens mit Hilfe von Stellgliedern durchgeführt wird.

5. Verfahren nach einem der vorhergehenden Ansprüche, wobei der Schritt des Scannens durch Bewegen der Objektivlinse durchgeführt wird.

6. Verfahren nach einem der vorhergehenden Ansprüche, wobei der Gewebeschnitt einen horizontal beabstandeten Schnitt, einen unter einem Winkel beabstandeten Schnitt oder einen vertikal beabstandeten Schnitt darstellt.

7. Verfahren nach einem der vorhergehenden Ansprüche, das ferner den Schritt des Umwandelns der Beleuchtung von der Quelle in zirkular polarisiertes Licht umfasst, damit die Gewebeprobe mit dem zirkular polarisierten Licht beleuchtet werden kann, das von der Objektivlinse fokussiert wird.

8. Verfahren nach Anspruch 7, wobei der Schritt des Sammelns den Schritt des Sammelns von zirkular polarisiertem, reflektiertem Licht von der beleuchteten Gewebeprobe umfasst, das rechtwinklig zu dem zirkular polarisierten Licht ist, mit dem die Gewebeprobe beleuchtet wurde, und/oder wobei vorzugsweise der Schritt des Umwandelns unter Verwendung einer Viertelwellenplatte durchgeführt wird.

9. Verfahren nach Anspruch 1, wobei das Verfahren ferner folgende Schritte umfasst: Bereitstellen eines Strahlteilers zum Aufnehmen der Beleuchtung von der Quelle unter einem schiefen Winkel zum Bereitstellen eines kreisrunden Strahls; und Polarisieren der Beleuchtung von dem Strahlteiler unter Verwendung einer Viertelwellenplatte zum Bereitstellen eines zirkular polarisierten Strahls, wobei der Schritt des Fokussierens ferner ein Fokussieren des zirkular polarisierten Strahls mit der Objektivlinse auf die Gewebeprobe umfasst, der Schritt des Aufnehmens ferner ein Aufnehmen des zurückgesandten Lichts umfasst, das auf die Viertelwellenplatte und dann auf den Strahlteiler fällt, und der Schritt des Erfassens ferner ein Erfassen eines reflektierten Teils des zurückgesandten Lichts umfasst, der von dem Strahlteiler auf einen Detektor fällt, der zurückgesandtes Licht über die Lochblende erfasst.

10. Verfahren nach Anspruch 9, wobei das zurückgesandte Licht, das von der Objektivlinse gesammelt wird, einen Anteil aufweist, der zirkular polarisiert rechtwinklig zu dem Strahl ist, der in die Gewebeprobe hinein fokussiert wird, und die Viertelwellenplatte den Anteil des zurückgesandten Lichts in linear polarisiertes rechtwinkliges Licht umwandelt, und der Strahlteiler, durch Reflektieren eines Teils des zurückgesandten Lichts, die Anteile aus dem zurückgesandten Licht herausfiltert.

11. System zum konfokalen Abbilden von Gewebeproben mit einer freiliegenden Oberfläche, umfassend:
ein Gehäuse, das so ausgelegt ist, dass es in der Hand gehalten wird, eine konfokale Abbildungsoptik in dem Gehäuse, wobei die konfokale Abbildungsoptik Folgendes umfasst:
ein Fenster, das an dem Gehäuse angebracht ist und eine Gewebekontaktfläche in einer direkten Druckkontaktbeziehung mit einer Oberfläche der Gewebeprobe definiert, damit eine stabile Betrachtungsposition aufrechterhalten wird, wenn das Gehäuse in der Hand gehalten wird und die Gewebeprobe von der Objektivlinse abgebildet wird, das Fenster eine Platte aus einem Material ist, das für die Beleuchtung durchlässig ist,
eine Objektivlinse des Mikroskops, die in der Lage ist, auf oder unter der freiliegenden Oberfläche zu fokussieren und zurückgesandtes Licht von der Gewebeprobe zu sammeln, Stellglieder in dem Gehäuse, die von elektrischen Signalen angesteuert werden, zum Scannen des Brennpunkts durch die Gewebeprobe, in der der Brennpunkt unter der freiliegenden Oberfläche verläuft,
einen Diodenlaser in dem Gehäuse, der die Gewebeprobe durch eine konfokale Abbildungsoptik beleuchtet,
eine Viertelwellenplatte in dem Gehäuse, die eine einfallende Polarisation von dem Diodenlaser umwandelt,
eine Lochblenden-Lichtdetektorbaugruppe in dem Gehäuse, die das zurückgesandte Licht aufnimmt, das von der Objektivlinse gesammelt wird, und das zurückgesandte Licht in elektrische Signale umwandelt,
ein Computersystem, das ein oder mehrere Bilder von Gewebeschnitten entsprechend den elektrischen Signalen liefert, die von der Lichtdetektorbaugruppe ausgegeben werden, und
eine Anzeige, die mit dem Computersystem gekoppelt ist, damit sie das eine oder die mehreren Bilder von Gewebeschnitten anzeigt und keine Biopsie zum Erkennen von Zellanomalien in der Gewebeprobe notwendig ist.

12. System nach Anspruch 11, das ferner Spiegel umfasst, die das zurückgesandte Licht optisch in die Lochblenden-Lichtdetektorbaugruppe einkoppeln.

13. System nach Anspruch 11, das ferner Mittel zum Verarbeiten der elektrischen Signale von der Lichtdetektorbaugruppe umfasst, damit das Computersystem in die Lage versetzt wird, das eine oder die mehreren Bilder zu liefern.

14. System nach Anspruch 11, wobei die konfokale Abbildungsoptik, die Lochblenden-Lichtdetektorbaugruppe und die Laserdiode in einer vereinheitlichten Bauweise in dem Gehäuse vorhanden sind.

15. System nach Anspruch 11, wobei das eine oder die mehreren Bilder von Gewebeschnitten jeweils einen horizontal beabstandeten Schnitt, einen unter einem Winkel beabstandeten Schnitt oder einen vertikal beabstandeten Schnitt darstellt bzw. darstellen.

16. System nach Anspruch 11, wobei die konfokale Viertelwellenplatte die Laserbeleuchtung in zirkular polarisiertes Licht umwandelt, das von der Objektivlinse auf die Gewebeprobe fokussiert wird, und die Objektivlinse das zirkular polarisierte, reflektierte Licht von der Gewebeprobe sammelt, das rechtwinklig zu dem zirkular polarisierten Licht ist, das von der Objektivlinse auf die Gewebeprobe fokussiert wurde.

17. System nach Anspruch 11, wobei die konfokale Abbildungsoptik Folgendes umfasst: einen Strahlteiler zum Aufnehmen der Laserbeleuchtung von der Laserdiode unter einem schiefen Winkel zum Bereitstellen eines kreisrunden Strahls, der von der Viertelwellenplatte polarisiert wird, damit ein zirkular polarisierter Strahl bereitgestellt wird, wobei die Objektivlinse so ausgelegt ist, dass sie den zirkular polarisierten Strahl fokussiert und Licht sammelt, das von der Gewebeprobe zurückgesandt wird, wobei das zurückgesandte Licht auf die Viertelwellenplatte und dann auf den Strahlteiler fällt, und der Strahlteiler einen Teil des zurückgesandten Lichts, das darauf fällt, reflektiert, und der reflektierte Teil des zurückgesandten Lichts optisch in die Lichtdetektorbaugruppe eingekoppelt wird.

18. System nach Anspruch 17, wobei das zurückgesandte Licht, das von der Objektivlinse gesammelt wird, einen Anteil aufweist, der zirkular polarisiert rechtwinklig zu dem Strahl ist, der in die Gewebeprobe hinein fokussiert wird, und die Viertelwellenplatte den Anteil des zurückgesandten Lichts in linear polarisiertes rechtwinkliges Licht umwandelt, und der Strahlteiler, durch Reflektieren eines Teils des zurückgesandten Lichts, die Anteile aus dem zurückgesandten Licht herausfiltert.

## Revendications

1. Procédé d'imagerie confocale d'un échantillon de tissu ayant une surface exposée, comprenant les étapes consistant à :
fournir un boîtier adapté pour être portatif possédant une source d'illumination laser et une lentille d'objectif ;
placer ledit échantillon de tissu contre une fenêtre pouvant être fixée audit boîtier définissant une surface de contact de tissu en relation de contact de pression directe avec la surface dudit échantillon de tissu, de manière à maintenir une position d'observation stable lorsque ledit échantillon de tissu est imagé par l'intermédiaire de ladite lentille d'objectif et que ledit boîtier est tenu à la main, et ladite fenêtre est une plaque de matériau transparent à ladite illumination ;
focaliser l'illumination avec ladite lentille d'objectif sur ou sous la surface exposée sur une tache focale ;
balayer la tache focale dans un plan à travers l'échantillon de tissu s'étendant sous ladite surface exposée ;
collecter la lumière retournée à partir dudit échantillon de tissu par l'intermédiaire de ladite lentille d'objectif ;
détecter la lumière retournée par l'intermédiaire d'une ouverture sténopéique, dans lequel ladite lumière retournée est couplée optiquement à l'ouverture sténopéique ;
former une image d'une section de tissu à partir de ladite lumière retournée détectée ; et afficher ladite image de ladite section de tissu pour éviter la nécessité d'une biopsie pour visualiser des anomalies cellulaires dans l'échantillon de tissu.

2. Procédé selon la revendication 1, dans lequel ladite étape de détection comprend en outre de convertir la lumière retournée en signaux électriques, et ledit procédé comprend en outre les étapes consistant à traiter lesdits signaux électriques pour fournir une image de ladite section de tissu.

3. Procédé selon l'une quelconque des revendications précédentes, dans lequel lesdites étapes de focalisation, de balayage, de collecte et de détection sont exécutées dans ledit boîtier.

4. Procédé selon l'une quelconque des revendications précédentes, dans lequel ladite étape de balayage est réalisée à l'aide d'actionneurs.

5. Procédé selon l'une quelconque des revendications précédentes, dans lequel ladite étape de balayage est effectuée en déplaçant ladite lentille d'objectif.

6. Procédé selon l'une quelconque des revendications précédentes, dans lequel ladite section de tissu représente l'une d'une section espacée horizontalement, d'une section espacée angulairement, ou d'une section espacée verticalement.

7. Procédé selon l'une quelconque des revendications précédentes, comprenant en outre l'étape consistant à convertir l'illumination de ladite source en lumière polarisée circulairement pour permettre audit échantillon de tissu d'être illuminé par ladite lumière polarisée circulairement focalisée par ladite lentille d'objectif.

8. Procédé selon la revendication 7, dans lequel ladite étape de collecte comprend l'étape consistant à collecter une lumière réfléchie polarisée circulairement à partir de l'échantillon de tissu illuminé qui est orthogonale à la lumière polarisée circulairement qui a illuminé l'échantillon de tissu, et/ou dans lequel, de préférence, ladite étape de conversion est effectuée en utilisant une lame quart d'onde.

9. Procédé selon la revendication 1, dans lequel ledit procédé comprend en outre les étapes consistant à :
fournir un diviseur de faisceau pour recevoir ladite illumination à partir de ladite source à un angle oblique pour fournir un faisceau circulaire ; et polariser ladite illumination à partir dudit diviseur de faisceau en utilisant une lame quart d'onde pour fournir un faisceau polarisé circulairement, dans lequel ladite étape de focalisation comprend en outre de focaliser ledit faisceau polarisé circulairement avec ladite lentille d'objectif sur l'échantillon de tissu, ladite étape de réception comprend en outre de recevoir ladite lumière retournée incidente sur ladite lame quart d'onde puis sur ledit séparateur de faisceau, et ladite étape de détection comprend en outre de détecter une partie réfléchie de ladite lumière retournée incidente à partir dudit diviseur de faisceau sur un détecteur qui détecte la lumière retournée par l'intermédiaire de ladite ouverture sténopéique.

10. Procédé selon la revendication 9, dans lequel ladite lumière retournée collectée par ladite lentille d'objectif a une composante qui est polarisée circulairement orthogonale au faisceau focalisé dans l'échantillon de tissu, et ladite lame quart d'onde convertit la composante de la lumière retournée en lumière orthogonale polarisée linéairement, et ledit diviseur de faisceau, en réfléchissant une partie de ladite lumière retournée, filtre lesdites composantes de ladite lumière retournée.

11. Système d'imagerie confocale d'échantillons de tissu ayant une surface exposée, comprenant :
un boîtier qui est adapté pour être portatif, une optique d'imagerie confocale dans ledit boîtier, ladite optique d'imagerie confocale comprenant :
une fenêtre fixée audit boîtier définissant une surface de contact de tissu en relation de contact de pression directe avec une surface dudit échantillon de tissu, de manière à maintenir une position d'observation stable lorsque ledit boîtier est tenu à la main et que ledit échantillon de tissu est imagé par ladite lentille d'objectif, ladite fenêtre est une plaque de matériau transparent à ladite illumination,
une lentille d'objectif dudit microscope et étant adaptée pour focaliser sur ou sous la surface exposée et collecter la lumière retournée à partir de l'échantillon de tissu,
des actionneurs dans ledit boîtier entraînés par des signaux électriques pour balayer ladite tache focale à travers l'échantillon de tissu, dans lequel ladite tache focale s'étend sous ladite surface exposée,
une diode laser dans ledit boîtier qui illumine l'échantillon de tissu à travers une optique d'imagerie confocale,
une lame quart d'onde dans ledit boîtier convertissant une polarisation incidente à partir de la diode laser,
un ensemble photodétecteur sténopéique dans ledit boîtier qui reçoit ladite lumière retournée collectée par ladite lentille d'objectif et convertit ladite lumière retournée en signaux électriques,
un système informatique qui fournit une ou plusieurs images de sections de tissu en fonction des signaux électriques délivrés par ledit ensemble photodétecteur, et
un affichage couplé audit système informatique pour afficher lesdites une ou plusieurs images de sections de tissu et qui évite la nécessité d'une biopsie pour détecter toute anomalie cellulaire dans l'échantillon de tissu.

12. Système selon la revendication 11, comprenant en outre des miroirs qui couplent optiquement la lumière retournée audit ensemble photodétecteur sténopéique.

13. Système selon la revendication 11, comprenant en outre un moyen pour traiter lesdits signaux électriques provenant dudit ensemble photodétecteur afin de permettre audit système informatique de fournir lesdites une ou plusieurs images.

14. Système selon la revendication 11, dans lequel ladite optique d'imagerie confocale, ledit ensemble photodétecteur sténopéique et ladite diode laser sont dans une construction unifiée dans ledit boîtier.

15. Système selon la revendication 11, dans lequel lesdites une ou plusieurs images de sections de tissu représentent chacune l'une d'une section espacée horizontalement, d'une section espacée angulairement, ou une section espacée verticalement.

16. Système selon la revendication 11, dans lequel ladite lame quart d'onde convertit ladite illumination laser en lumière polarisée circulairement qui est focalisée par ladite lentille d'objectif sur l'échantillon de tissu, et ladite lentille d'objectif collecte ladite lumière réfléchie polarisée circulairement à partir de l'échantillon de tissu qui est orthogonale à la lumière polarisée circulairement qui a été focalisée par ladite lentille d'objectif sur l'échantillon de tissu.

17. Système selon la revendication 11, dans lequel ladite optique d'imagerie confocale comprend : un diviseur de faisceau pour recevoir ladite illumination laser à partir de ladite diode laser à un angle oblique pour fournir un faisceau circulaire qui est polarisé par ladite lame quart d'onde pour fournir un faisceau polarisé circulairement, dans lequel ladite lentille d'objectif est adaptée pour focaliser ledit faisceau polarisé circulairement et pour collecter la lumière retournée à partir dudit échantillon de tissu, ladite lumière retournée étant incidente sur ladite lame quart d'onde puis sur ledit diviseur de faisceau, et ledit diviseur de faisceau réfléchit une partie de ladite lumière retournée incidente sur celui-ci, et ladite partie réfléchie de ladite lumière retournée est couplée optiquement audit ensemble photodétecteur.

18. Système selon la revendication 17, dans lequel ladite lumière retournée collectée par ladite lentille d'objectif a une composante qui est polarisée circulairement orthogonale au faisceau focalisé dans l'échantillon de tissu, et ladite lame quart d'onde convertit la composante de la lumière retournée en lumière orthogonale polarisée linéairement, et ledit diviseur de faisceau, en réfléchissant une partie de ladite lumière retournée, filtre lesdites composantes de ladite lumière retournée.
